# EUROPEAN PATENT APPLICATION

(11) **EP 1 622 057 A1**
(43) Date of publication of application: **01.02.2006**
(21) Application number: 04425559.4
(22) Date of filing: 26.07.2004
(51) Int. Cl.: G06F 19/00

(54) **Method and system for providing home-healthcare and information and communication technology platform therefor**

(71) Applicant: Siemens S.p.A., 20126 Milano (IT)
(72) Inventor: Palazzi, Patrizia, 20154 Milano (IT)
(74) Representative: Giustini, Delio

(57) **Abstract**

A system for of providing home-healthcare interventions to a set of patients by means of mobile operators, includes:
- a set of mobile terminals (40) for use by the operators in performing said assistance interventions,
- a central server (100) having associated a data base (100a) for collecting data items concerning the patients and said interventions,
- a communication network (35) between the mobile terminals (40) and the central server (100),
- the mobile terminals (40) equipped with application modules enabling them to run autonomously the assistance events, and
- a communication mechanism over the network (35) ensuring, at least in a discontinuous manner, communication between the mobile terminals (40) and the central server (100) to permit exchange of data therebetween, thus ensuring synchronization of information available with the mobile terminals (40) and the central server (100).

## Description

### Field of the invention

The present invention relates to the field of home-healthcare activities and in particular of home-palliative care. The present invention has been developed with particular attention paid to its possible use in managing activities performed in support of so-called "terminal" patients.

Exemplary of these are the activities performed by the social-healthcare organization VIDAS of Corso Italia, 17 - Milan (Italy).

### Description of the related art

Home-healthcare activities performed by social-healthcare organizations generate every day a large number of data on patients. Today this data is mostly managed through paper forms, which are filled in every day by the various operators at the end of the activities of intervention. The data are then manually recorded in a database, in which both the patient information and the operator information reside. These data can normally be consulted at the headquarters of the social-healthcare organization.

While these activities has been mostly provided by resorting to the conventional structures of home-healthcare organizations, a growing trend may be traced in the art aiming at increasingly supporting such activities by automated tools such as e.g. computerized information systems.

A general survey of this topic is provided e.g. by Eric Verkaar in "The Development of a Computerized Information System for Integrated Home Care in The Netherlands", co-published simultaneously in Computers in Human Services (the Haworth Press, Inc.) vol. 12, No. 3/4, 1995, pp 273, 287 and Human Services in the Information Age (Ed. Jackie Rafferty, Jan Steyaert and David Colombi) the Haworth Press, Inc., 1995, pp 273-287.

Additional information on that subject can be found in:
- L. Ruffino, L. "Using a Pocket PC to implement telemedicine for homecare", Proceedings of the 2001 Symposium on Applications and the Internet Workshops, San Diego, CA, USA 8-12 Jan.2001, IEEE Comput. Soc: Los Alamitos, CA, USA, p.129;
- N. Bricon-Souf et al., "A helpful framework for the organization of the homecare", 2001 Conference Proceedings of the 23^{rd} Annual International Conference of the IEEE Engineering in Medicine and Biology Society, Istanbul, Turkey, 25 - 28 Oct. 2001, Piscataway, NJ, USA, pp. 3567-3570, vol. 4;
- Way Da-Ming "A homecare service support system using the mobile Internet and Java technology" in Journal of Shanghai University 2001, vol. 5, No. SUP, pp. 177-80, and also in JP 2003281281-A.

Additionally, a particularly extensive patent literature exists in the area of so-called telemedicine systems, especially for distance monitoring of patients suffering from heart-related diseases.

### Object and summary of the invention

Despite certain appreciable advantages, most solutions considered so far fail to meet the specific needs of the home-healthcare activities, especially as regards the type of patients hinted at in the introductory portion of this description.

The object of the invention is thus to provide an improved arrangement adapted to satisfy these specific needs, while dispensing with the intrinsic drawbacks of the prior art arrangements discusses in the foregoing.

According to the present invention, that object is achieved by means of a method having the features set forth in the claims that follow. The invention also relates to a corresponding system and a related Information and Communication Technology platform for performing the steps of the method of the invention

A preferred embodiment of the invention relies on a computer system operated in the Application Service Provider (ASP) mode, which will enable real-time and on-site recording of the clinical data and of the data for management of the Patient especially as regards patients receiving Palliative Care.

The aims of this service are multiple:
- providing the operators, in real-time at the Patient's home, with access to data concerning the Patient and the Patient's family, with the possibility of immediate updating;
- providing a tool that will enable exchange of information between the operators;
- acquiring data useful for processing and measurement of indicators, in particular process indicators, aimed at enabling actions of control; and
- reducing/replacing paper forms in order to favour a "paperless" process, handled by a computer system.

According to a preferred embodiment of the invention, all the operators are equipped with a mobile terminal, through which they can gain access to the service. In that way, the organization they belong to will be equipped with a tool for communication, consultation, and clinical monitoring of performance.

Responsibility for management of the central components of the computer system (server and archive) may be external to the organization; in that way, the social-healthcare organization may use the service as user, without being concerned with dedicating time and resources to operative maintenance of the computer system.

### Brief description of the annexed drawings

The invention will now be described, by way of non-limiting example, with reference to the annexed figures of drawing, where:
- Figure 1 shows an exemplary architecture of the system described herein;
- Figure 2 shows a detailed exemplary architecture of the system described herein;
- Figure 3 shows an example of wireless communication within the system of Figure 1;
- Figure 4 shows an example of flow of activity in the system described herein; and
- Figure 5 shows a typical interaction pattern the system described herein.

### Detailed description of exemplary embodiments of the invention

By way of general introduction to a detailed description of an exemplary embodiment of the invention, it is worthwhile to recall that social-healthcare organization currently operate at the patient home through different professional roles, namely:
- the Physician
- the Professional Nurse
- the Social Worker
- the Physiotherapist
- the Social-Healthcare Auxiliary

or other relevant professional roles, organized in teams. In general, it is the Physician and above all the Nurse who activate the other operators of the team, each with different responsibilities in regard to the Patient. An individual inter-professional plan of healthcare is defined for each Patient.

In addition to care of a social and health nature provided at the home of the patient, the organization usually also sees to providing support to the Patient family, both through health education and by means of psychological underpinning.

Skills and activities thus diversified in regard to patients, the clinical and psychological conditions of whom change very rapidly, must be managed in a coordinated, adequate and timely way. This is why all the services offered are managed in a centralized way at the organization headquarters.

The above management entails a system of day-to-day monitoring of intervention in order to guarantee a verification of the activities and a correct economic evaluation.

As regards the quality indicators and in particular the process indicators, at present there are available only a few indications regarding procedures recommended by Evidence-Based Medicine (EBM) and Evidence-Based Nursing (EBN). There still remains, however, the question regarding the definition of national and international standard indicators for the operative processes of home healthcare.

If, on the one hand, the data on the activities are recorded on paper or electronic media, on the other, the need for indicators does not regard so much the collection of purely quantitative results as the processing of said data via correlation, with the use of the human and material resources employed to achieve said results. The intention is, in other words, to assess the effectiveness, efficiency and appropriateness of the activities performed, in clinical, organizational and economic terms.

More specifically, in the class of social-healthcare organization, organizations such as VIDAS have existed and exist thanks to the contribution of partners, bodies, firms, and private individuals that enable financing of its activities.

This model of operation, which enables structural independence and autonomy of management, is based upon the communication and transparency in regard both to the subjects who support the organization and to those who benefit therefrom. There consequently appears clearly the need for tools that will facilitate collection and handling of information for the purposes of said communication.

A computer-based system as described herein guarantees the operators access to the information, data and communications regarding patients and provide a tool for monitoring the quality indicators, which will enable verification of the adequacy and appropriateness of the healthcare interventions.

As seen previously, the operators who interact with the patients may be distinguished in different categories (Physician, Professional Nurse, andsoon) and to each category there corresponds a user profile within the system.

A further profile exists, namely, that of the Decision Maker, who must have the possibility from fixed terminals (152) or mobile devices (40) of displaying and exporting the results of the measurement of the indicators in a management and control perspective.

The system must moreover enable recording of information on another role who is of fundamental importance in healthcare activities, namely, the "Care Giver", i.e., the patient relative who is to share and be involved in management of the care that the patient receives. For this purpose, the system envisages recording the level of compliance of the Care Giver.

The system must guarantee automatic recognition of the data-access device (palm-top terminal, PC or other devices) irrespective of the user profile.

Furthermore, the system must enable printing of paper documentation corresponding to the objective examination of the patient. Since said information must remain at the home of the patient, each operator is provided with a portable printer.

Hence the system is:
- a tool for clinical monitoring and monitoring of performance;
- a tool for communication (at present formalized via a verbal or on-paper request); and
- a tool for consultation (evidence data base).

The information gleaned and updated will be processed by the system in order to measure the quality indicators. The information will be obtained directly by the monitoring system on-site and real-time, so that "a posteriori" entry of information will not be necessary.

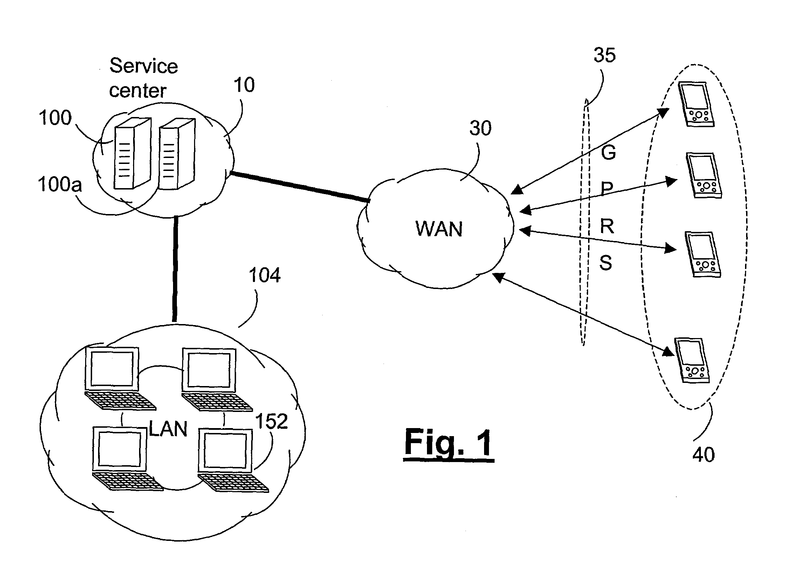

An exemplary architecture of a preferred embodiment of the arrangement described herein is shown in figure 1.

In order to better understand the principles underlying the arrangement described herein, one has to keep in mind that the health service represents quite a specific sector.

Information Technology (IT) makes available "vertical" Computer Systems which may be used for the management of social-health activities. However, their effective use tends to be limited by an inadequate approach on the part of the users and on the part of technical and administrative managers thereof. Furthermore, a clinical IT system is a live and dynamic system in so far as IT is an area that undergoes fast evolution which entails a rapid obsolescence, and the social-health activities that it manages also themselves undergo continuous variations.

The arrangement described herein overcomes these obstacles by giving the opportunity of establishing a deep-seated partnership between provider and client. Such a partnership is preferably achieved with the ASP model.

The Application Service Provider can be understood as being constituted by third parties who manage and distribute to customers from a remote central site services and software solutions through a wide-area data transmission network (WAN, Wide Area Network).

From the technological standpoint, in ASP mode, the applications are not installed at the client sites, but hosted at the service provider and accessible to the clients via telematic connection. This configuration centralizes management of the services and of the information and reduces the necessary technological resources, as well as the costs of management that must be sustained by the clients.

The exemplary arrangement described hereinafter corresponds to an ASP HomeCare Service developed in view of the specific needs of the VIDAS organization.

The architecture of the system envisages an ASP remote service centre, where there are installed the central components of the computer system, namely, the server and archives. The end users can use the Service by connecting up via wide-area lines to the ASP centre with mobile terminals, e.g. palm-top terminals (via wireless communication technology, e.g. GPRS) or else with standard PC workstations of users enabled for using the service.

The wide-area data-transmission lines must be safe, reliable, dedicated, and highspeed.

Functionally, setting-up of an efficient communication network involves the purchase of network equipment, identified in firewalling systems, for protection of the managing server.

A service centre 10, including an application server 100 and an associate database server 100a is connected to Social-healthcare Organization 104 whose activities are supported by one or more terminals 152, belonging to the Social-healthcare Organization local area network (LAN). Reference 30 designates a wide area network (WAN) that allows a number of remote terminals, preferably in the form of mobile terminals 40 generally distributed over the area of activity of the organization, to establish a connection to the service centre 10 via the network 30. This preferably occurs by resorting to a mobile communication network operating according to a wireless communication standard e.g. GPRS, as indicated in 35.

Coverage of each and every terminal 40 by the wireless network 35 may be discontinuous in that a given terminal 40 may, at least temporarily, be outside the network coverage. This may be due to a number of factors such as, e.g. the specific location where the terminal is temporarily located being not covered by the network or the terminal being somewhat "shielded" by a building infrastructure.

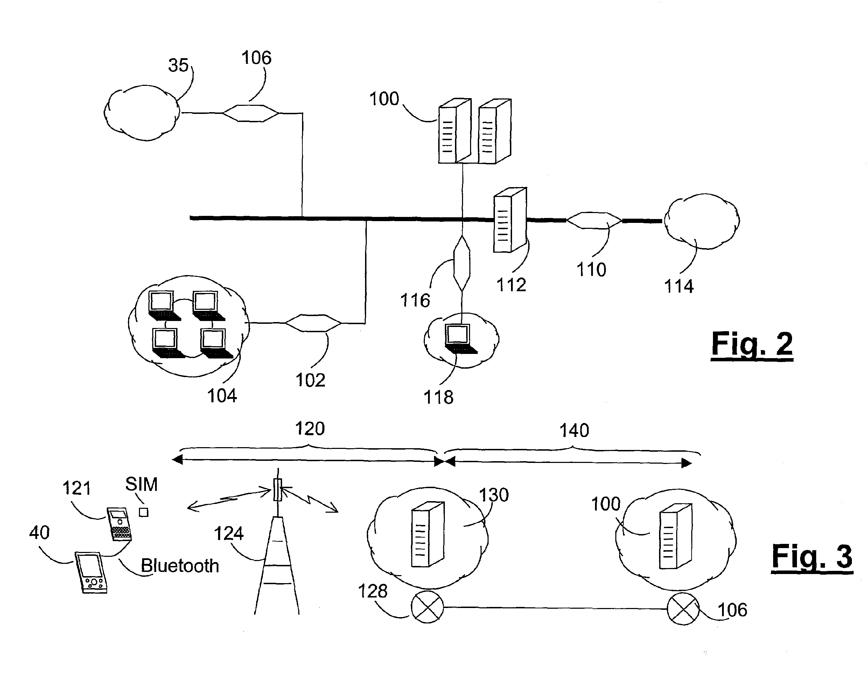

The block diagram of figure 2 is exemplary of a typical implementing architecture of a system as described herein.

The application (ASP) server 100 is connected by means of a gateway 102 to the local area network 104 (see also figure 1). Preferably, the gateway 102 is a dedicated channel gateway.

The server 100 is connected to the (public) wireless network 35 by means of another gateway 106. To allow an eventual server (100) connection with the Internet (114), reference 110 designates a further gateway, having necessary associated a firewall module 112. Additionally, another gateway 116 connects the server 100 with a remote-assistance centre 118.

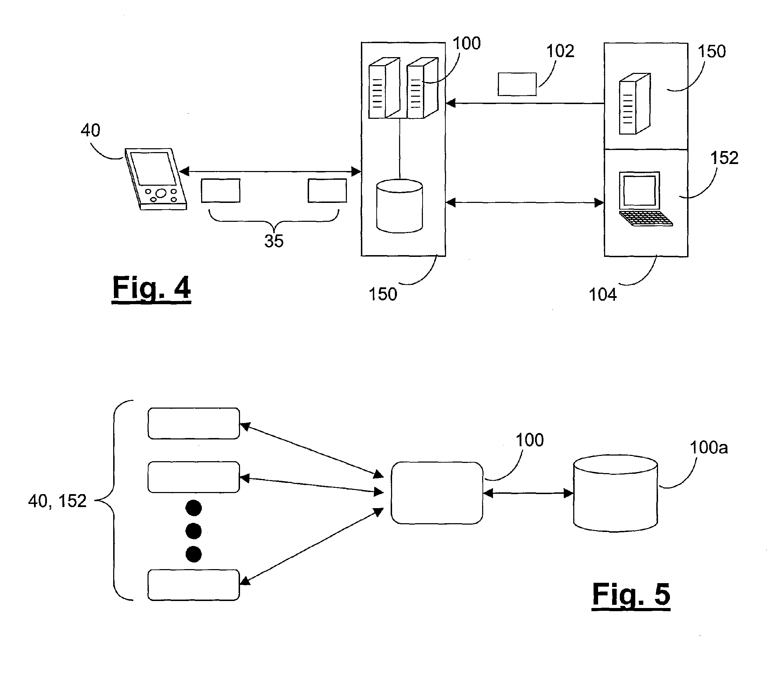

The block diagram of Figure 3 further details the criteria adopted for communication between the server 100 and the mobile terminals 40 via the wireless network. Adopting such a connection guarantees characteristics of security and quality of service (QoS) in communication between the devices 40 and the server 100 by implementing tunnelling, authentication and dedicated link arrangements between the server 100 and the nearest point of presence (PoP) 130 of the wireless network.

Transport via the wireless network adds the intrinsic advantages related to wireless access to the flexibility of packet traffic management having regard to the increased request for mobility.

The users are granted exclusive access to the service via a pool of subscribed identity card e.g. a Subscribed Identity Module (SIM) card. Any attempt at connecting with the system from an un-registered subscribed identity card is aborted during the set-up phase of the wireless connection, at the level of the Packet Data Protocol (PdP) context 120, by preventing traversing the access network.

This function is ensured by provisioning on the user's subscribed identity card a dedicated Access Point Name (APN). Therefore, from the logical view point, remote users are offered totally dedicated transmission chain.

The wireless data session from a mobile device 40 takes place by means of a direct or indirect (e.g. via Bluetooth to a mobile phone 121) connection to the wireless network via a wireless backbone 124.

The access and authentication procedure through the subscribed identity card takes place simultaneously with the activation of the wireless session.

Reference 140 designates an Layer 2 Tunneling Protocol (L2TP) tunnel implemented between the point of presence (PoP) 130 of the wireless network and the server 100 (which is located with the seat of the organization providing the home-healthcare).

Data are routed between the PoP router 128 and the router 106 associated with the server 100.

Any remote client is allotted a private IP address belonging to the local network (LAN) 104 of the seat of the organization following authentication on the server 100 by means of an A.A.A. (authentication, authorization, accounting) protocol, e.g. the RADIUS protocol.

Specific steps are preferably taken at the hardware level in order to ensure operational security.

In order to ensure a maximum level of continuity in the service provided, the hardware installed with the service centre 10 (namely the server 100 and the related archive) usually includes a pair of twin servers.

Power supply of the servers is via Uninterruptible Power Supplies (UPS) Groups ensuring system operation even in the case of power failure (black-out). Management of the system also typically provides for periodical data back-up (e.g. via DAT devices) for data saving and recovery (Disaster Recovery) in the case of irreversible loss.

At the level of application software, security and privacy are ensured against fraudulent access via typical user-name/password authentication procedures whereby only the authorised users are admitted to the system.

Additionally, different profiles are usually contemplated depending on the role played by a given user. In the case of those users that operate "in-the-field", data collected are certified by means of digital signatures by resorting to personal smart cards. This approach is significant as it provides legal value to the visits to the patients while ensuring that the object signed are not tempered with.

Data security, privacy and integrity at the level of data storage and network transport are ensured by encryption mechanisms and SSL (Secure Socket Layer) / TLS (Transport Layer Security) protocols.

Further security is provided at the language level. The applications executed at each node in the architecture (including the mobile clients 40) are implemented via programming language, e.g. Java Language. Security of the application in that language is ensured by a certain number of protection mechanism that are intrinsic of the programming language. Exemplary of these are: automatic memory management; range checking on strings and arrays; code verification by the compiler.

The kind of service provided contemplates various modes of exchanging sensitive data between the server 100 and the users 40. Proper security is ensured for each of these modes.

In order to permit exchange of data between the server 100 and the local network 104 a dedicated digital circuit connection properly sized for data traffic management is preferably used. In addition to providing a satisfactory transmission bandwidth and avoiding perturbation related to analogue signal processing (reliability), this arrangement has the additional advantage of ensuring a permanent and exclusive link between the respective locations, without any risks of direct intrusions by third parties.

The users' mobile computers 40 can access the server 100 via the wireless network 35 only after authentication e.g. via the RADIUS protocol or other A.A.A. protocol. Connection between the server 100 and the point of presence (PoP) of the wireless network is again preferably provided by means of a digital dedicated circuit.

Management and update interventions on the server 100 are preferably effected remotely via authorised personnel via a remote connection permitting access to the server via a password and an authentication key.

The server 100 is configured for eventual accessing the Internet 114 (see figure 2) to permit on-line updating of system patches.

A digital signature mechanism is used for authenticating messages, transactions and documents of any kind transmitted within the system. This permits to render evident and verify the origin and integrity of a given document.

As indicated, within the framework of a system as considered herein it is important to be in a position to ensure jointly the medical-legal value, the privacy and security of the reports generated by the various operating units.

The digital signature is an authentication system derived from the typical instruments of cryptography. It is in a position to ensure the following security functions:
- authenticity of the data origin,
- data integrity,
- impossibility to repudiate the data.

A digital signature is based on the use of a pair of "asymmetric cipher keys". Specifically, two cipher keys are used, a direct one and an inverse or reciprocal one. The keys used for encrypting data cannot be used for decrypting them. Additionally, knowledge of one of the two keys does not provide any information on the other key. The two keys, one of which is public while the other is private, essentially implement two complementary functions, whereby any processing performed by means of one of the keys can be properly interpreted only by means of the other key. The owner of the pair of keys makes his public key available while maintaining secret the private key.

In the case of the home-healthcare service considered herein, a basic need arises as regards permitting the operator to provide a certain type of assistance to patients to sign a corresponding electronic document recording the kind of assistance provided.

Such document, once stored on the terminal device (for instance a mobile computer 40) is not per se confidential: it must be attributed with certainty to the operator (physician, professional nurse, social worker, physiotherapist, social-healthcare auxiliary) who performed the related activity.

The operator ciphers the document by means of his or her private key.

Any (authorised) party having or wishing to verify the document can do that by reading the document by using the operator's public key.

This operating arrangement based on a digital signature mechanims duly takes into account the fact that if an operator A may decrypt a message by using the public key of an operator B, it is important to make sure that only this latter may have encrypted the message by using his or her secret key.

A digital signature ensures the integrity of the message sent, the authenticity of the data source, an the possibility for such a source not to be repudiated (that is the sender/source of the message can be identify in such a way that it is not possible for him or her to deny having created that specific message).

A problem thus arises in making sure that a public key does really and univoquely belong to the person indicated thereby. In fact, it may happen that a fraudulent user may present himself or herself to the system as a legitimate user and receive by the other users, that are not aware of this, confidential documents/information.

The public key certificate, which represents a sort of digital passport, provides a solution. Such a certificate, which, i.a. includes the user's public key, is signed by a trusted entity authorised to perform such an operation. The certification authority ensures that a certain public key belongs to a given subject. Following certain verifications, such an authority may grant a certificate in the form of a signed message (encrypted by means of its public key) including the public key of the subject in question.

The owner himself or herself may even ignore the private key loaded (stored) in devices such as magnetic cards or smart cards (which is the current case, e.g. of automatic bank teller cards).

Within the framework of the service considered herein, the users (operators) are provided with a personal smart card including a microprocessor and a smart card reader to be inserted into the compact flash slot of the mobile device. Once the data related to the intervention just performed are saved (stored) on the mobile device, the application running thereon requests that the Postal Index Number (PIN) code for the card has an input. If the respective operation is correctly performed, the digital signature procedure of the document is started.

At the subsequent data synchronisation step with the server 100, a digitally signed document (which corresponding to a report having a full medical/legal value) is sent to the server 100 to be stored in the database 100a associated therewith.

According to a preferred embodiment, the mobile terminal 40 from which each operator will gain access to the service has the following minimum requirements: an Intel PXA250 processor, a 64-MB RAM, and the possibility to connect to a wireless network directly or indirectly (e.g. through a connection to a mobile phone). In addition, in order to guarantee security of the data, the mobile terminal 40 is provided with a memory card, in which is automatically archived a back up copy of the data. Other minimum functions of the mobile terminal are: the wireless connection to PC desktops, to printing devices (portable printers) and to smart card readers for the digital signature.

The service described herein includes a series of activities each involving an exchange of data between the components of the system architecture: the mobile devices 40, the server 100, the terminals 152 within the LAN 104 of the organization.

The block diagram of Figure 4 details the respective data flows with specific reference to the interaction modes between the various components.

Specifically, the function of block diagram of Figure 4 encompasses a number of possible events.

A first event concerns communication of data between the server 100 and the mobile devices 40 via the wireless network. This event includes:
i) downloading from the server 100 the updated data concerning the patients followed, new patients being admitted to the service, communications and documents.
ii) uploading in the server 100 the updated data concerning the home-healthcare assistance provided with the associated communication and requests.

The two operations are performed in parallel during the data synchronisation step and are usually completed in an interval of e.g. 30 seconds.

Another typical event concerns the automatic and continuous updating of the information available with the server 100 concerning the following facts: deaths, assistance being discontinued, hospitalisation, planning of attendance tasks, new patients being admitted to the system, association between patients and operator teams.

These data are currently recorded with a server 150 used by the organization for the internal management of systems activities. Typically, those variations that have occurred in the meantime are simultaneously sent to the server 100 (which is connected via a dedicated digital link).

A third event may relate to data downloading/uploading essentially as described previously in connection with interactions between the server 100 and the mobile devices 40: however, in this case data exchange involves server 100 and a fixed terminal 152. The workstation 152 is connected to the LAN 104 by exploiting the dedicated digital link with the server 100. Typically the synchronization operation requires about 10 seconds to be completed.

The system just described by way of example is based upon Web technology and corresponds to the general architecture shown in Figure 5. The components shown in Figure 5 correspond to the basic components of a typical Client/Server application (Two-Tier applications), constituted by a client (e.g. the terminals 40, 152) that implements the user interface with functions of processing and communication, and by a server (here the ASP server 100), which supplies a series of services, such as management and access to the data of a database (here the database 100a).

The architectural elements are basically of four different types: Application server, Database server, organization server, organization client.

In the arrangement described herein at the site of the service provider there is the Application Service Provider (ASP) core, constituted by the Application server 100 and by the database 100a.

The applications (referred to as Client applications) on each mobile device 40 or fixed terminal 152 will be installed for consultation and entry of examinations.

The Client applications on the mobile device serve as application interface for the operators, offering the functions of:
- consulting the informative material coming from the evidence data bank;
- entering any modifications of data on patient examinations;
- sending requests for aids, medical facilities and appliances, prostheses and resources corresponding to the patient examined;
- reading and writing data on the patient.

The Application server 100 offers the functions of:
- answering the requests of the operators who, via a respective mobile equipment 40 and through the wireless network 35, send and receive the data on the examinations;
- synchronizing between the mobile equipment 40 and the Database server those activities related to patients associated to the operator, and last examination made on each patient;
- invoking the service of synchronization, upon request by an operator or else periodically, of personal data on patients and operators between the Database server and the organization server;
- sending the requests for aids, medical facilities and appliances, prostheses and resources to the operators enabled to fulfil the requests;
- calculating the measurements requested by the quality indicators and offering services of consultation of said indicators;
- managing the evidence data bank.

The server supporting the database 100a has the function of:
- filing the data on the examinations and the digitally signed documents;
- keeping a copy of personal data on patients and operators, updated to the last synchronization with the organization server;
- historicizing the trends in time of the indicators;
- keeping the evidence data bank.

The organization server 150 for the application infrastructure created will be put in a condition to offer the main functions of synchronizing personal data on patients and operators between the organization server and the ASP server (in particular the Database server).

Consequently, without prejudice to the underlying principles of the invention, the details and the embodiments may vary, also appreciably, with reference to what has been described by way of example only, without departing from the scope of the invention as defined by the annexed claims.

## Claims

1. A method of providing home-healthcare interventions to a set of patients by means of mobile operators, the method including the steps of:
- providing:
- a set of mobile terminals (40) for use by said operators in performing said assistance interventions,
- a central server (100) having associated a data base (100a) for collecting data items concerning said patients and said interventions,
- a communication network (35) between said mobile terminals (40) and said central server (100), and
- application modules in said mobile terminals (40) enabling said mobile terminals (40) to run autonomously said assistance events, and
- ensuring, at least in a discontinuous manner, communication between said mobile terminals (40) and said central server (100) to permit exchange of data therebetween.

2. The method of claim 1, **characterised in that** it includes the step of providing a security mechanism in communication between said mobile terminals (40) and said central server (100).

3. The method of claim 2, **characterised in that** it includes the step of associating with said communication network (35) an access control procedure including user authentication.

4. The method of claim 3, **characterised in that** it includes the steps of:
- selecting, as said communication network, a network (35) requesting the presence of a subscribed identity card (121) associated with the mobile user terminals, and
- associating (121) a subscribed identity card with each said mobile terminal (40).

5. The method of either of claims 1 or 4, **characterised in that** it includes the steps of:
- providing said communication network in the form of a mobile telephone network (35), and
- associating with each said mobile terminal (40) a mobile phone (121) configured for communication over said mobile telephone network (35).

6. The method of any of claims 1, 4 or 5, **characterised in that** said communication network is a wireless network (35).

7. The method of claim 1, **characterised in that** it includes the step of implementing a digital signature mechanism to ensure authenticity, integrity and non repudial of data entered by said operators in the respective mobile terminals (40).

8. The method of claim 7, **characterised in that** it includes the steps of providing said mobile operators with smart cards carrying cryptographic information for implementing said digital signature mechanism.

9. The method of either of claims 7 or 8, **characterised in that** it includes the step of associating with said digital signature mechanism the issue of digital certificates for said mobile operators.

10. The method of any of the previous claims, **characterised in that** said communication between said mobile terminals (40) and said central server (100) includes the steps of:
- downloading from said central server (100) towards said mobile terminals (40) of information concerning said set of patients, and
- uploading from said mobile terminal (40) towards said central server (100) of updated data concerning said assistance intervention performed.

11. The method of any of the previous claims, **characterised in that** it includes the step of configuring said central server (100) for operation according to an ASP paradigm.

12. The method of any of the previous claims, **characterised in that** it includes the step of connecting to said central server (100) some fixed terminals (152) for permitting remote management of said home-healthcare.

13. The method of claim 12, **characterised in that** it includes the steps of providing a dedicated link between said central server (100) and said management terminals (152).

14. The method of any of the previous claims, **characterised in that** it includes the step of providing each said mobile terminal (40) with a respective operator application profile, said profile including a respective defined set of interactions allowed between said mobile terminal (40) and the operator, said profile being differentiated for different types of operators.

15. The method of any of the previous claims, **characterised in that** it includes the step of selecting said mobile terminals (40).

16. A system for of providing home-healthcare interventions to a set of patients by means of mobile operators, the system including:
- a set of mobile terminals (40) for use by said operators in performing said assistance interventions,
- a central server (100) having associated a data base (100a) for collecting data items concerning said patients and said interventions,
- a communication network (35) between said mobile terminals (40) and said central server (100),
- said mobile terminals (40) equipped with application modules enabling said mobile terminals (40) to run autonomously said assistance events, and
- a communication mechanism over said network (35) ensuring, at least in a discontinuous manner, communication between said mobile terminals (40) and said central server (100) to permit exchange of data therebetween.

17. The system of claim 16, **characterised in that** it includes a security mechanism in the communication between said mobile terminals (40) and said central server (100).

18. The system of claim 17, **characterised in that** it includes, associated with said communication network (35), an access control procedure including user authentication.

19. The system of claim 18, **characterised in that** it includes:
- as said communication network, a network (35) requesting the presence of a subscribed identity card(121) associated with the mobile user terminals, and
- a subscribed identity card associated (121) with each said mobile terminal (40).

20. The system of either of claims 16 or 19, **characterised in that** it includes:
- said communication network in the form of a mobile telephone network (35), and
- a mobile phone (121) configured for communication over said mobile telephone network (35) associated with each said mobile terminal (40).

21. The system of any of claims 16, 19 or 20, **characterised in that** said communication network is a wireless network (35).

22. The system of claim 16, **characterised in that** it includes a digital signature mechanism to ensure authenticity, integrity and non repudial of data entered by said operators in the respective mobile terminals (40).

23. The system of claim 22, **characterised in that** it includes, provided to said mobile operators or other authorized operators, smart cards carrying cryptographic information for implementing said digital signature mechanism.

24. The system of either of claims 22 or 23, **characterised in that** it includes, associated with said digital signature mechanism, digital certificates issued for said mobile operators.

25. The system of any of the previous claims 16 to 24, **characterised in that** it includes said mobile terminals (40) and said central server (100) configured for performing the the steps of:
- downloading from said central server (100) towards said mobile terminals (40) of information concerning said set of patients, and
- uploading from said mobile terminal (40) towards said central server (100) of updated data concerning said assistance intervention performed.

26. The system of any of the previous claims 16 to 25, **characterised in that** it includes said central server (100) for operation according to an ASP paradigm.

27. The system of any of the previous claims 16 to 26, **characterised in that** it includes, connected to said central server (100), some fixed terminals (152) for permitting remote management of said home-healthcare.

28. The system of claim 27, **characterised in that** it includes a dedicated link between said central server (100) and said management terminals (152).

29. The system of any of the previous claims 16 to 28, **characterised in that** it includes the Decision Maker profile for supervising the data coming from the said central server (100), regardless of the kind of terminal device

30. The system of any of the previous claims 16 to 29, **characterised in that** it includes each said mobile terminal (40) equipped with a respective operator application profile, said profile including a respective defined set of interactions allowed between said mobile terminal (40) and the operator, said profile being differentiated for different types of operators.

31. The system of any of the previous claims 16 to 30, **characterised in that** it includes said mobile terminals (40).

32. An Information and Communication Technology platform able to perform the method of any of claims 1 to 16.
